# EUROPÄISCHE PATENTSCHRIFT

(11) **EP 2 064 127 B1**
(45) Veröffentlichungstag und Bekanntmachung des Hinweises auf die Patenterteilung: **19.05.2010**
(21) Anmeldenummer: 07801673.0
(22) Anmeldetag: 16.08.2007
(51) Int. Cl.: B65D 51/00, B65D 53/04, A61J 1/14, A61M 5/28

(54) **VERSCHLUSSSYSTEM FÜR BEHÄLTNISSE**
CLOSING SYSTEM FOR CONTAINERS
SYSTÈME DE BOUCHAGE POUR RÉCIPIENTS

(30) Priorität: 31.08.2006 DE 102006040888
(43) Veröffentlichungstag der Anmeldung: 03.06.2009
(73) Patentinhaber: LTS LOHMANN Therapie-Systeme AG, 56626 Andernach (DE)
(72) Erfinder: HOFFMANN, Hans-Rainer, 56566 Neuwied (DE); MATUSCH, Rudolf, 35041 Marburg (DE)
(74) Vertreter: Schmidt, Werner
(86) Internationale Anmeldenummer: PCT/EP2007/007211
(87) Internationale Veröffentlichungsnummer: WO 2008/025455

(56) Entgegenhaltungen:
- DE-U1- 9 011 309
- FR-A- 2 373 454
- US-A- 3 001 658

## Beschreibung

Die Erfindung betrifft ein Verschlusssystem für Behältnisse zur Aufbewahrung oder Verabreichung von flüssigen, pastösen oder pulverförmigen Stoffen, das aus einer - mit einem Durchgangsloch ausgestatteten - Kappe und einem Verschlusselement besteht, wobei die Kappe das Verschlusselement auf dem Behältnis im Bereich der zu verschließenden Öffnung mittels eines am Behältnis vorhandenen Hintergriffs kraft- und/oder formschlüssig hält.

Aus der DIN ISO 11 040 Teil 3 aus dem Jahr 1993 sind Aluminium-Bördelkappen für Dentalkarpulen bekannt. Mit diesen Bördelkappen werden Glaszylinder z.B. mittels Kolbenstopfen oder Dichtscheiben fest verschlossen. Dazu werden nach dem Aufsetzen der Kolbenstopfen bzw. dem Auflegen der Dichtscheibe die Bördelkappen auf dem Glaszylinder durch einen Umformvorgang an der Bördelkappe fixiert. Im Teil 2 der Norm werden u.a. dünne Dichtscheiben beschrieben, die aus einem elastomeren Werkstoff bestehen.

Aus der DE 90 11 309 U1 ist eine Flasche für die Nieren - Dialyse bekannt. Die Flasche hat einen Schraubdeckel mit einer zentralen großen Bohrung. Zwischen dem Schraubdeckel und dem oberen stirnseitigen Gewinderand der Flasche ist eine dünne, mittels einer Kanüle leicht durchstechbare Kunststoffscheibe angeordnet.

Aus der DE 696 02 171 T2 ist eine Vorrichtung zum Versiegeln von Behälteröffnungen durch Anbringen einer Kunststoffstreckfolie bekannt. Beschrieben wird ein Rahmen, in dem eine Streckfolie eingespannt ist. Die im Rahmen gehaltene Streckfolie wird mittig auf eine zu verschließende Flaschenöffnung gelegt. Anschließend wird der Rahmen in Richtung Flaschenhals gepresst, so dass sich die Streckfolie durch ihre Materialdehnung am oberen Bereich des Flaschenhalses, die Flaschenöffnung dicht verschließend, anlegt.

Der vorliegenden Erfindung liegt daher die Problemstellung zugrunde, ein Verschlusssystem für Behältnisse zu entwickeln, das ohne Eintragen von Wärmeenergie ein maschinell einfaches, viren-, bakterien- und sporendichtes und dauerhaft sicheres Verschließen des Behältnisses ermöglicht.

Diese Problemstellung wird mit den Merkmalen des Hauptanspruches gelöst. Dazu weist die die Behältnisöffnung umgebende Stirnfläche, auf der das Verschlusselement aufliegt, eine Vertiefung auf. Das Verschlusselement ist eine viren-, bakterien- und sporendichte Folie, die auf der Stirnfläche und zumindest bereichsweise über der Vertiefung liegt. Beim Aufsetzen der Kappe ist zwischen dem Verschlusselement und der Vertiefung ein Elastomer- oder Klebstoffring angeordnet, der die Vertiefung ausfüllt.

Alternativ liegt das Verschlusselement auf einer mit einer Erhöhung ausgestatteten Stirnfläche auf. Dabei ist am Verschlusselement ein Elastomerring angeordnet, in den sich beim Aufsetzen der Kappe die Erhöhung eindrückt.

Des Weiteren gibt es ein Verschlusssystem, bei dem die Kappe an der dem Verschlusselement zugewandten Kappeninnenseite mindestens eine - federnd auf dem Verschlusselement aufliegende - geschlossene, elastische Dichtlippe aufweist.

Ferner ist auch ein Verschlusssystem denkbar, das ohne eine Kappe auskommt. In diesem Fall liegt der Elastomerring, der mit dem Verschlusselement festhaftend verbunden ist, radial verspannt an einer, z.B. im Bereich der zu verschließenden Öffnung gelegenen, radialen Außenkontur des Behältnisses an.

Mit der Erfindung wird ein einfach und sicher zu handhabendes Verschlusssystem vorgestellt, mit dem schonend flüssige, pastöse oder pulverförmige Stoffe, insbesondere Medikamente, wie z.B. eine Proteine enthaltende Arzneiflüssigkeit, in einem Behältnis dicht dauerhaft eingeschlossen werden.

Weitere Einzelheiten der Erfindung ergeben sich aus den Unteransprüchen und den nachfolgenden Beschreibungen schematisch dargestellter Ausführungsformen.
- Figur 1:: Medikamentenkammer mit Kappe und Dichtfolie;
- Figur 2:: vergrößerter Teilschnitt zu Figur 1;
- Figur 3:: wie Figur 2, jedoch ohne Führungskonus;
- Figur 4:: wie Figur 2, jedoch mit rinnenförmiger Vertiefung zur Aufnahme des Elastomerrings;
- Figur 5:: Karpule mit Kappe und Dichtfolie;
- Figur 6:: Medikamentenkammer mit einer gegenüber der Dichtfolie elastischen Kappe; ,
- Figur 7:: Ausschnitt zu Figur 5;
- Figur 8:: wie Figur 5, jedoch mit mindestens einem anderen Folienandrückelement;
- Figur 9:: wie Figur 4, jedoch mit Randerhöhung und flachem Elastomerring;
- Figur 10:: Medikamentenkammer mit Spannring;
- Figur 11:: Medikamentenkammer ohne Spannring und ohne Kappe.

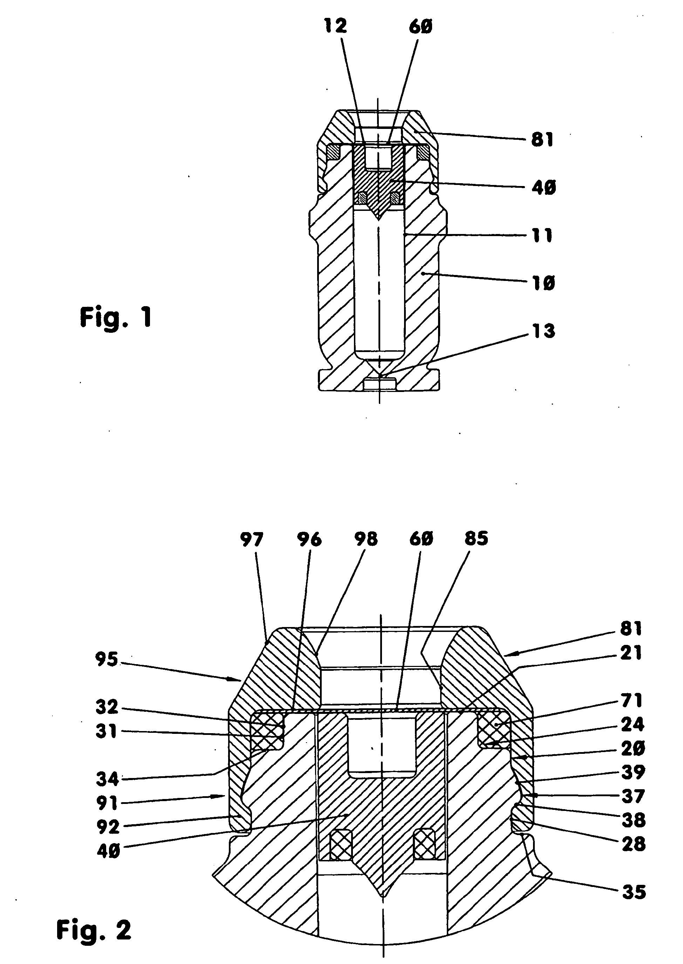

Die Figur 1 zeigt ein Behältnis (10) mit einem Kolben (40) und einem Verschlusssystem. Das Behältnis (10) wird im Weiteren als Medikamentenkammer bezeichnet.

Die Medikamentenkammer (10) ist in Kombination mit dem Kolben (40) eine Baugruppe eines Injektionssystems. In der Medikamentenkammer (10) wird das zu verabreichende Medikament beispielsweise in flüssiger Form zunächst gelagert. Dazu muss das Medikament im Zylinder (11) der Kammer (10) viren-, bakterien- und sporendicht eingeschlossen werden. Der beispielsweise konische Zylinder (11) hat zwei Öffnungen (12, 13). Eine vordere Öffnung (13) ist die Austrittsdüse. Eine hintere Öffnung (12) dient u.a. dem Befüllen des Zylinders (11). Im hinteren Bereich des Zylinders (11) ist bei befüllter Kammer (10) der Kolben (40) positioniert. Unmittelbar hinter dem Kolben (40) befindet sich eine Kappe(81), die mit Hilfe eines Verschlusselements (60) die hintere Öffnung (12) viren-, bakterien- und sporendicht verschließt. Selbstverständlich kann das Behältnis (10, 50) auch nur mit einer, also der mit dem vorgestellten Verschlusssystem verschlossenen, Öffnung (12) ausgestattet sein. Ggf. kann das Verschlusssystem auch gasdicht sein.

Zur Fixierung der elastischen Kappe (81) hat der hintere Bereich der Kammer (10), die beispielsweise aus dem Kunststoff COC gefertigt ist, eine bestimmte Kontur, vgl. Figur 2. Die Kammer (10) hat in erster Näherung ein zylinderrohrförmiges Endstück (20) mit einer beispielsweise planen Stirnfläche (21) und einer zylindrischen Außenkontur (28). Das Endstück (20) endet vorn in einem Gehäusebund (35).

Im Übergangsbereich zwischen der Stirnfläche (21) und der zylindrischen Außenkontur (28) befindet sich ein weiterer Gehäusebund (31). Die Breite dieses Gehäusebundes (31) beträgt z.B. 50% der Wandstärke des Endstücks (20). Die Tiefe dieses Gehäusebundes (31) ist geringfügig größer als dessen Breite. Die radiale Flanke (32) des Gehäusebunds (31) ist zylindrisch, während die axiale Flanke (34) plan ist.

Unterhalb des Gehäusebundes (31) befindet sich eine beispielsweise zweiflankige, umlaufende Rasterhöhung (37). Ihre Längsausdehnung entspricht ca. 50% der zwischen den beiden Gehäusebünden (31, 35) gelegenen Endstücklänge. Die beispielsweise dort mittig sitzende, umlaufende Rasterhöhung (37) zeigt im Ausführungsbeispiel keine Unterbrechung. Die Rasterhöhung (37) hat eine vordere (38) und eine hintere Flanke (39). Der hintere Flankenwinkel beträgt z.B. 17 ± 3 Winkelgrade, während der vordere Flankenwinkel z.B. 51 ± 3 Winkelgrade beträgt. Im Bereich der Kontaktzone der beiden Flanken (38, 39) ist die Rasterhöhung (37) abgerundet.

Nach Figur 2 sitzt auf der hinteren Stirnseite (21) des Endstücks (20) das Verschlusselement (60) und ein Elastomerring (71). Das Verschlusselement (60) ist beispielsweise eine 0,15 Millimeter dicke - aus einer Aluminiumlegierung gefertigte - Dichtungsfolie. Das Folienmaterial kann auch Tyvek^{®}, PE, PET oder ein Verbundmaterial sein. Die Dichtungsfolie (60) ist eine runde, flache und flexible Scheibe. Der Elastomerring (71) ist beispielsweise aus Silikon-, Chlor- oder Butylkautschuk hergestellt. Er ist ein geschlossener Ring mit einem zumindest annähernd runden Einzelquerschnitt. In der Zone, über die er an der Dichtungsfolie (60) angeklebt oder anvulkanisiert ist, ist er abgeflacht ausgeführt.

Die Kappe (91) besteht aus zwei Abschnitten, einem Befestigungsbereich (91) und einem Bodenbereich (95). Der Befestigungsbereich (91) ist ein im Wesentlichen zylinderrohrförmiger Abschnitt. Er umgreift das Endstück (20) im Bereich der dortigen Rasterhöhung (37). Seine Innenkontur ist genau so geformt, dass sie nach der Montage der Kappe (91) zumindest im Bereich der Flanke (38) spielfrei auf dem Endstück (20) sitzt. Die Innenkontur liegt an der anderen Flanke (37) nicht oder nur bereichsweise an.

Der Bodenbereich (95), der nach Figur 2 bereichsweise mit einer planen Bodenfläche (96) auf der Dichtungsfolie (60) aufliegt, hat eine kegelstumpfmantelförmige Außenkontur (97) und eine trichterförmige, zentrale Ausnehmung (98). Die zentrale Ausnehmung (98), die dem Durchgangsloch (85) entspricht, hat einen kleinsten Durchmesser, der geringfügig kleiner ist als der Durchmesser der hinteren Öffnung (12) des Zylinders (11). Der an der Bodenfläche (96) anschließende Bereich der Ausnehmung (98) ist zylindrisch geformt. Der restliche Bereich der Ausnehmung (98) weitet sich nach hinten trichterförmig auf. Diese Trichterform unterstützt das Einfädeln eines - hier nicht dargestellten - Stößels, über den der Kolben (40) im Zylinder (11) beim Auslösen des Injektors bewegt wird.

Nach einem Befüllen der Medikamentenkammer (10) und einem Einsetzen des Kolbens (40) wird auf die Stirnfläche (21) der Kammer (11) die Dichtungsfolie (60) zusammen mit dem Elastomerring (71) aufgesetzt. Der an der Dichtungsfolie (60) festhaftende Elastomerring (71) umgreift dabei zentrierend die radiale Flanke (32) des Gehäusebundes (31). Beim Aufsetzen der Kappe (81) gleitet diese mit ihren Hintergriff (92) über die Rasterhöhung (37). Sobald der Hintergriff (92) an der vorderen Flanke (38) anliegt, hat die Kappe (81) ihre Endlage erreicht. Dann liegt die Bodenfläche (96) fest auf der Dichtungsfolie (60) auf. Gleichzeitig sitzt der Elastomerring (71) im Bereich des Gehäusebundes (31) abdichtend zwischen der Kappe (81) und dem Endstück (20). In axialer Richtung ist der Elastomerring (71) zwischen der axialen Flanke (34) und der fest an der Bodenfläche (96) angepressten Dichtungsfolie (60) eingezwängt. Die Klemmkraft der Kappe (81) wird hier beispielsweise mittels der Ringspannkraft des Befestigungsbereichs (91) erzeugt.

Der Gehäusebund (35) dient bei der Montage der Kappe (81) als Hilfsanschlag.

Besonders bei kleinen Behältnissen ist es auch denkbar, dass die Kappe das gesamte Behältnis umgreift. Hierbei verrastet dann die Kappe an dem als Rastelement (37) wirkenden Boden des Behältnisses.

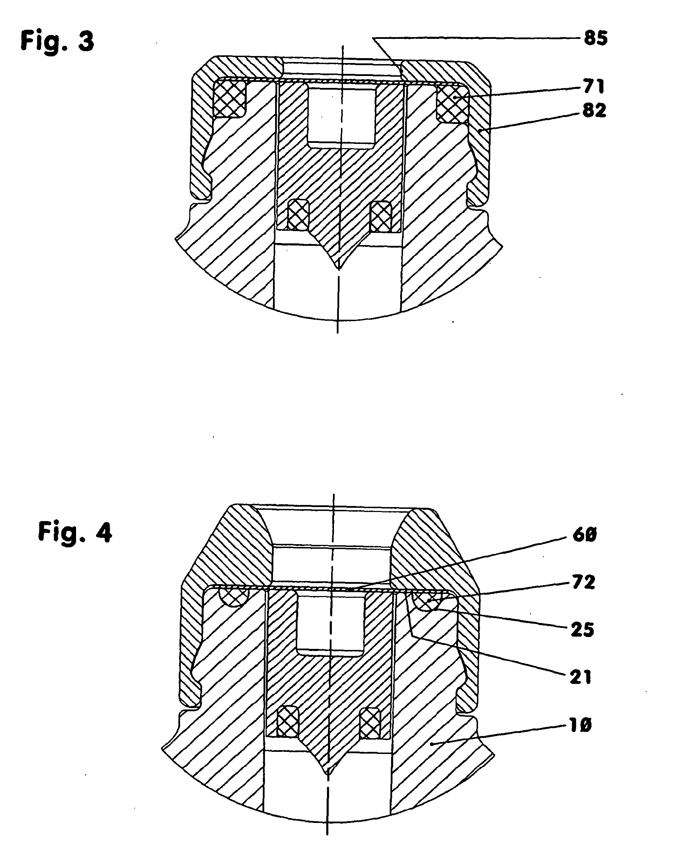

Die Figur 3 zeigt ein Verschlusssystem mit einer vereinfachten Kappe (82). Dieser Kappe (82) fehlt der Einfädelungstrichter (98).

In der Figur 4 ist eine weitere Variante zu Figur 2 dargestellt. Bei dieser Variante befindet sich in der Stirnfläche (21) eine radial eingearbeitete Vertiefung (25) in Form eines Ringkanals. Der Ringkanal (25), dessen Einzelquerschnitt sich aus einer Rechteckfläche und einer Halbkreisfläche zusammensetzt, nimmt den an der Dichtungsfolie (60) befestigten Elastomerring (72) auf. Beidseits des Ringkanals (25) befinden sich jeweils geschlossen Bereiche der Stirnfläche (21). In diesen Bereichen liegt die Dichtungsfolie (60) fest an der Stirnfläche (21) der Kammer (10) an.

Ggf. kann bei dieser Variante statt eines Elastomerrings (60) in den Ringkanal (25) ein Klebstoff eingebracht werden. Letzterer verklebt dann die aufgelegte Dichtungsfolie (60) mit der Kammer (10).

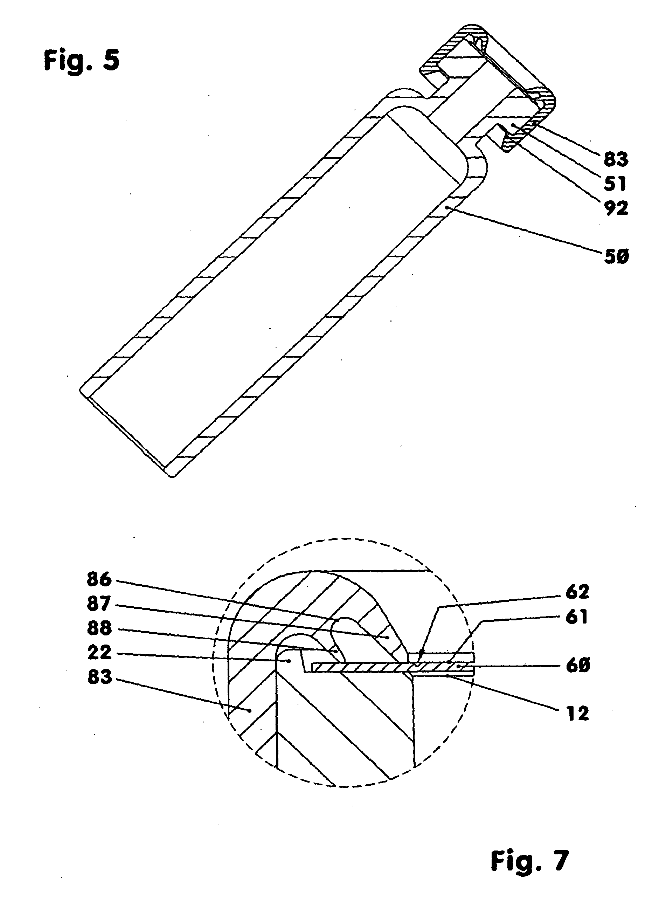

Statt einer Medikamentenkammer kann auch eine Glasflasche oder eine Karpule (50), vgl. Figur 5, verwendet werden. Hier umgreift eine Kappe (83) den Kragen (51) der Karpule (50) formschlüssig. Der Hintergriff (92) hat dabei im Einzelquerschnitt die Form eines Widerhakens.

Bei der Karpule (50) wie auch bei der Medikamentenkammer (10) kann die Stirnfläche (21) nach außen abfallend gestaltet sein. Demnach, beschreibt die Stirnfläche (21) z.B. einen Kegelstumpfmantel, der beispielsweise einen Spitzenwinkel von 158 ± 4 Winkelgraden aufweist. Die gedachte Kegelspitze liegt dabei außerhalb der Karpule (50) bzw. außerhalb der Medikamentenkammer (10).

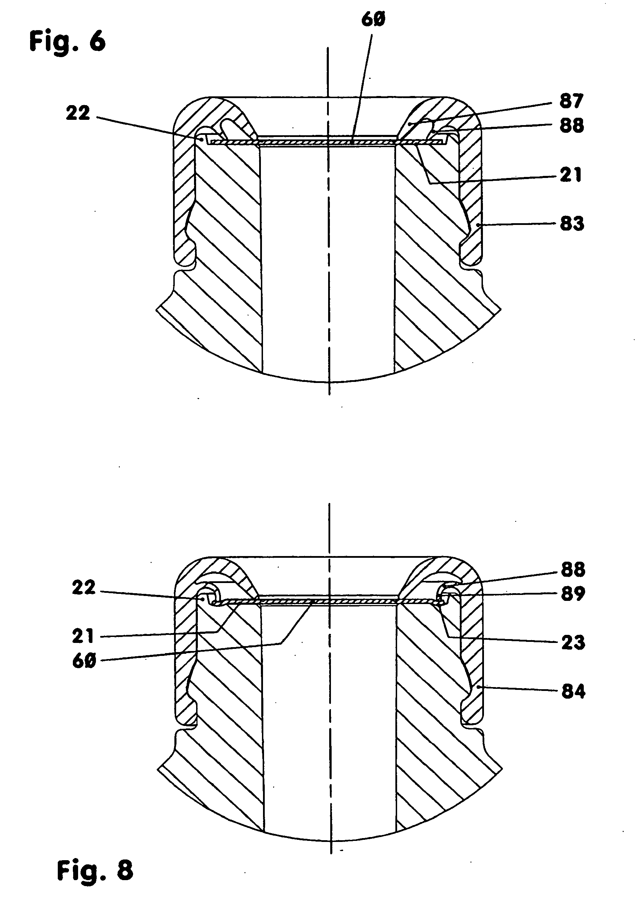

In Figur 6 ist ein Verschlusssystem dargestellt, das keinen Elastomerring benötigt. Stattdessen hat die Kappe (83) beispielsweise zwei Dichtlippen (87, 88),die die Dichtungsfolie (60) elastisch gegen die Stirnseite (21) der Medikamentenkammer (10) pressen, vgl. auch Figur 7. Die erste, äußere Dichtlippe (87) ist der Umgebung zugewandt. Sie presst die Dichtungsfolie (60) im Randbereich der Öffnung (12) gegen die Stirnfläche (21). Im Einzelquerschnitt ist die Dichtlippe (87) um ca. 45 Winkelgrade gegen die Dichtungsfolie (60) geneigt. Ihre Wandstärke ist hier größer als die der zweiten, inneren Dichtlippe (88).

Die zweite, innere Dichtlippe (88) sitzt geschützt unterhalb der ersten Dichtlippe (87). Sie drückt den äußeren Randbereich der Dichtungsfolie (60) gegen die Stirnfläche (21). Auch sie ist um ca. 45 Winkelgrade gegen die Dichtungsfolie (60) geneigt. Beide Dichtungslippen (87, 88) sind aus einem dauerelastischen Material gefertigt.

Damit bei der Montage des Verschlusssystems die Dichtungsfolie (60) zumindest weitgehend zentriert auf der Stirnfläche (21) aufgelegt werden kann, hat diese Variante einen Anschlagrand (22), dessen Höhe mindestens der zweifachen Folienstärke der Dichtungsfolie (60) entspricht.

In Figur 7 ist eine Ausschnittsvergrößerung zu Figur 6 dargestellt. In dieser Vergrößerung ist zu erkennen, dass die Dichtungsfolie (60) im Bereich des Randes der Öffnung (12) auf der Oberseite (61) eine Umlaufsicke (62) in Form einer Kerbe hat. Die Umlaufsicke (62) stellt eine mechanische Schwächung der Dichtungsfolie (60) dar. Sie soll es erleichtern, die Folie (60) beim Verabreichen des Medikaments aufzureißen.

Die Figur 8 zeigt eine Variante zu Figur 6. Die innere Dichtlippe (88) und die Stirnseite (21) haben hier eine andere Gestalt. Die Stirnseite (21) hat nach Figur 8 eine entlang dem Anschlagrand (22) verlaufende Rinne (23). In diese Rinne (23) wird beim Aufsetzen der Kappe (84) die Dichtungsfolie (60) mit Hilfe der inneren Dichtlippe (88) gepresst. Letztere ist im Einzelquerschnitt im Wesentlichen winkelförmig gestaltet, sodass die vordere Bereich (89) der Dichtlippe (88) nahezu senkrecht auf die Dichtungsfolie (60) drückt. Der hintere Bereich der Dichtlippe (88) dient als Federzone.

Beim Aufsetzen der Kappe (84) drückt die innere Dichtlippe (88) die Dichtungsfolie (60) in die Rinne (23). Dabei wird die Dichtungsfolie (60) gespannt, so dass sie eben anliegt.

Ggf. kann der vordere Bereich (89) der Dichtlippe (88) auch nach außen weisen. In diesem Fall ist der vordere Bereich Teil eines Kegelmantels, dessen Spitze oberhalb der Oberseite (61) der Dichtungsfolie (60) auf der verlängerten Mittellinie der Kammer (10) liegt. Hier kann beispielsweise auf die Rinne (23) verzichtet werden.

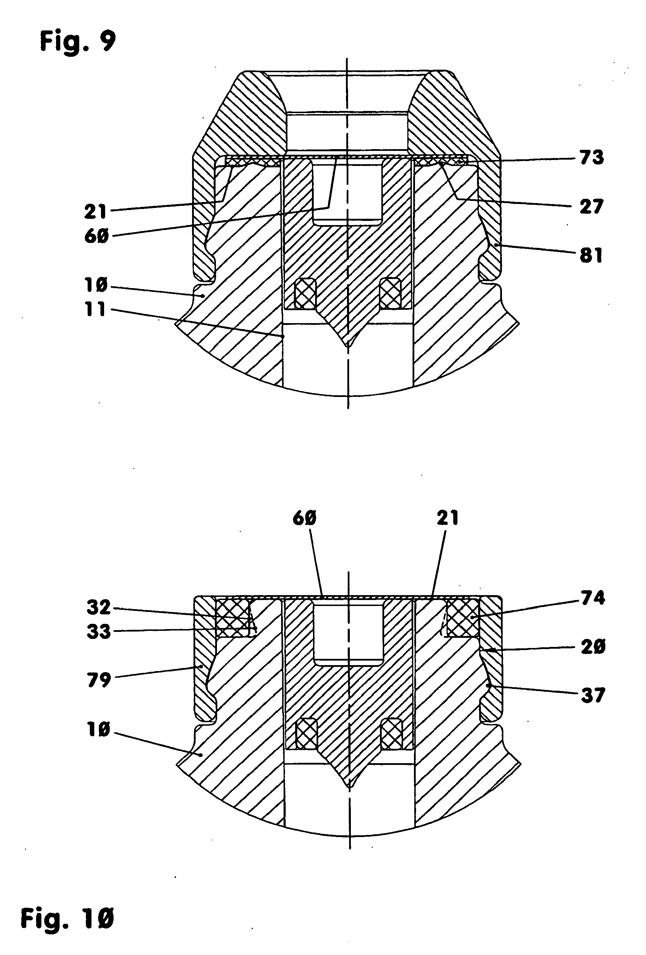

In Figur 9 ist ein Verschlusssystem dargestellt, das an der Stirnfläche (21) der Kammer (10) oder Karpule (50) statt einer umlaufenden Vertiefung eine Erhöhung (27) aufweist. Die Erhöhung (27) liegt zumindest annähernd mittig in der Ringfläche der Stirnfläche (21). Nach Figur 9 wird die Erhöhung (27) beispielsweise aus zwei Kegelstumpfmantelflächen gebildet, die sich im Einzelquerschnitt als Dreieck darstellen. Die Erhöhung kann nahezu jeden beliebigen Einzelquerschnitt haben. Ggf. liegen mehrere Erhöhungen auch nebeneinander. Ferner braucht die einzelne Erhöhung nicht konzentrisch zur Mittellinie der Kammer (10) angeordnet sein. Zudem muss sie in der Draufsicht auch nicht rund sein.

Auf der Stirnfläche (21) liegt die Dichtungsfolie (60) mit dem an ihr festhaftend angeordneten Elastomerring (73) an. Der Elastomerring (73) hat die Form einer gelochten Scheibe. Seine Materialstärke ist in der komprimierten Einbaulage mindestens um 30% größer als die Höhe der Erhöhung (27). Der Elastomerring (73) reicht hier bis an die Wandung des Zylinders (11) heran. Bei dieser Variante wird die Klemmkraft der Kappe (81) über die Elastizität des Elastomerrings (73) bestimmt.

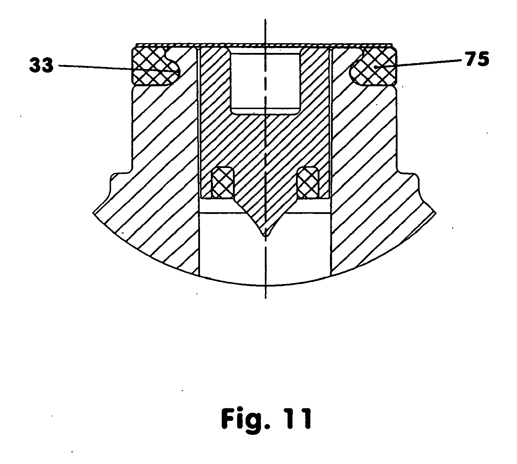

Die Figuren 10 und 11 zeigen eine Verschlusssystemvariante, die ohne Kappe auskommt. Diese Lösung basiert auf einer radialen Spannkraft des entsprechenden Elastomerrings (74, 75). Der Elastomerring (74, 75), der an der Unterseite der elastischen Dichtungsfolie (60) fest haftet, hat im nicht eingebauten Zustand einen kleineren mittleren Durchmesser. Erst bei der Montage wird er unter elastischem Aufdehnen auf das Endstück (20) und dort z.B. auf die radiale Flanke (32, 33) aufgesetzt.

Die radiale Flanke kann hierbei eine zylindrische (32) oder eine nichtzylindrische Kontur (33) haben. In Figur 10 ist die Kontur (33) gestrichelt dargestellt. Ihr Durchmesser nimmt ausgehend von der Stirnfläche (21) nach unten hin linear ab. Demnach hat die Flanke (33) die Form eines Kegelstumpfmantels, dessen theoretische Spitze beispielsweise im mittleren Bereich der Kammer (10) liegt.

Nach Figur 10 wird der Elastomerring (74) durch einen Spannring (79) radial gestützt und verspannt. Der Spannring (79) liegt dabei sowohl am Elastomerring (74) als auch an der Außenkontur (28) des Endstücks (20) an. Nur beispielhaft ist er hier über die Rasterhöhung (37) fixiert. Der Spannring (79) schließt zur Kammerrückseite hin mit der Dichtungsfolie (60) bündig ab. Ggf. umgreift der Spannring (79) nur den Elastomerring (74).

In Figur 11 wird ein Verschlusssystem gezeigt, bei dem die zwischen der Kontur (33) und dem Elastomerring (75) gelegene Montagefuge einen Hintergriff ausbildet. Ggf. hat der Elastomerring (75) schon im nicht eingebauten Zustande eine an die Kontur (33) angepasste Gegenform. Hier wird z.B. auf einen Spannring verzichtet.

Es sind auch Lösungen möglich, bei denen die Varianten aus den Figuren 1, 4, 6, 8, 10 und 11 zumindest bereichs- oder teilweise miteinander überlagert werden.

Selbstverständlich können die Dichtungsfolien (60) und ggf. auch die Elastomerringe (71-73) in den Bereichen mit denen sie die Stirnfläche (21) und die Vertiefungen (24, 25) oder Erhö hungen (27) kontaktieren, selbstklebende, virendichte Beschichtungen aufweisen.

Bezugszeichenliste:
- 10: Behältnis, Medikamentenkammer
- 11: Zylinder
- 12: Öffnung, hinten; Behälteröffnung
- 13: Öffnung, vorn; Austrittsdüse

- 20: Endstück
- 21: Stirnfläche
- 22: Anschlagrand
- 23: Rinne
- 24: Vertiefung am Rand
- 25: Vertiefung, mittig; Ringkanal
- 27: Erhöhung
- 28: Außenkontur, zylindrisch
- 31: Gehäusebund
- 32: radiale Flanke, zylindrisch
- 33: radiale Flanke, nicht zylindrisch
- 34: axiale Flanke

- 35: Gehäusebund, Hilfsanschlag

- 37: Rastelement, Rasterhöhung
- 38: vordere Flanke
- 39: hintere Flanke

- 40: Kolben

- 50: Behältnis, Karpule
- 51: Kragen

- 60: Verschlusselement, Dichtungsfolie
- 61: Oberseite
- 62: Sicke, Umlaufsicke

- 71: Elastomerring nach Figur 2 und 3
- 72: Elastomerring, Klebstoffring nach Figur 4
- 73: Elastomerring nach Figur 9
- 74: Elastomerring nach Figur 10
- 75: Elastomerring nach Figur 11

- 79: Spannring

- 81: Kappe nach Figur 2
- 82: Kappe, flach nach Figur 3
- 83: Kappe, mit Dichtlippen nach Figur 6
- 84: Kappe, mit Dichtlippen nach Figur 8

- 85: Durchgangsloch
- 86: Kappeninnenseite
- 87: Dichtlippe, außen
- 88: Dichtlippe, innen
- 89: vorderer Bereich

- 91: Befestigungsbereich
- 92: Hintergriff

- 95: Bodenbereich
- 96: Bodenfläche
- 97: Außenkontur
- 98: Ausnehmung, trichterförmig, Einfädelungstrichter

## Patentansprüche

1. Verschlusssystem für Behältnisse zur Aufbewahrung oder Verabreichung von flüssigen, pastösen oder pulverförmigen Stoffen, das aus einer - mit einem Durchgangsloch (85) ausgestatteten - Kappe (81, 82) und einem Verschlusselement (60) besteht, wobei die Kappe (81, 82) das Verschlusselement (60) auf dem Behältnis (10, 50) im Bereich der zu verschließenden Öffnung (12) mittels eines am Behältnis (10, 50) vorhandenen Rastelements (37) kraft- und/oder formschlüssig hält,
**dadurch gekennzeichnet,**
- **dass** die die Behältnisöffnung (12) umgebende Stirnfläche (21), auf der das Verschlusselement (60) aufliegt, eine Vertiefung (24, 25) aufweist,
- **dass** das Verschlusselement (60) eine viren-, bakterien- und sporendichte Folie ist, die auf der Stirnfläche (21) und zumindest bereichsweise über der Vertiefung (24, 25) liegt und
- **dass** beim Aufsetzen der Kappe (81, 82) zwischen dem Verschlusselement (60) und der Vertiefung (24, 25) ein Elastomer- oder Klebstoffring (71, 72) angeordnet ist, der die Vertiefung (24, 25) ausfüllt.

2. Verschlusssystem nach Anspruch 1,
**dadurch gekennzeichnet,**
**dass** das Verschlusselement (60) eine scheibenförmige Folie ist.

3. Verschlusssystem nach Anspruch 2,
**dadurch gekennzeichnet,**
**dass** die Folie (60) eine Materialstärke von 50 bis 300 Mikrometer hat.

4. Verschlusssystem nach Anspruch 2,
**dadurch gekennzeichnet,**
**dass** die Folie (60) aus einer Aluminiumlegierung hergestellt ist.

5. Verschlusssystem nach Anspruch 2,
**dadurch gekennzeichnet,**
**dass** die Folie (60) im Bereich der Öffnung (12) des Behältnisses (10, 50) auf ihrer Oberseite (61) eine die Folie (60) schwächende Sicke (62) aufweist.

6. Verschlusssystem nach Anspruch 1,
**dadurch gekennzeichnet,**
**dass** die Vertiefung (24) am äußeren Rand der Stirnfläche (21) liegt.

7. Verschlusssystem für Behältnisse zur Aufbewahrung oder Verabreichung von flüssigen, pastösen oder pulverförmigen Stoffen, das aus einer - mit einem Durchgangsloch (85) ausgestatteten - Kappe (83, 84) und einem Verschlusselement (60) besteht, wobei die Kappe (83, 84) das Verschlusselement (60) auf dem Behältnis (10, 50) im Bereich der zu verschließenden Öffnung (12) mittels eines am Behältnis (10, 50) vorhandenen Rastelements (37) kraft- und/oder formschlüssig hält,
**dadurch gekennzeichnet,**
- **dass** das Verschlusselement (60) eine viren-, bakterien- und sporendichte Folie ist und
- **dass** an der dem Verschlusselement (60) zugewandten Kappeninnenseite (86) mindestens eine - federnd auf dem Verschlusselement (60) aufliegende - geschlossene, elastische Dichtlippe (87, 88) angeordnet ist.

8. Verschlusssystem nach Anspruch 7,
**dadurch gekennzeichnet,**
**dass** an der Kappeninnenseite (86) eine innere (88) und eine äußere Dichtlippe (87) angeordnet ist.

9. Verschlusssystem nach Anspruch 8,
**dadurch gekennzeichnet,**
**dass** bei mindestens einer federnden Dichtlippe (88) der die Dichtungsfolie (60) kontaktierende Bereich - die Dichtungsfolie (60) spannend - nach außen orientiert ist und dabei im Einzelquerschnitt einem Winkel von 30 bis 60 Winkelgrade gegenüber der Mittelinie des Behältnisses (10, 50) einschließt.

10. Verschlusssystem für Behältnisse zur Aufbewahrung oder Verabreichung von flüssigen, pastösen oder pulverförmigen Stoffen, das aus einer - mit einem Durchgangsloch (85) ausgestatteten - Kappe (81, 82) und einem Verschlusselement (60) besteht, wobei die Kappe (81, 82) das Verschlusselement (60) auf dem Behältnis (10, 50) im Bereich der zu verschließenden Öffnung (12) mittels eines am Behältnis (10, 50) vorhandenen Rastelements (37) kraft- und/oder formschlüssig hält,
**dadurch gekennzeichnet,**
- **dass** die die Behältnisöffnung (12) umgebende Stirnfläche (21), auf der das Verschlusselement (60) aufliegt, eine die Behälteröffnung (12) umgebende Erhöhung (27) aufweist,
- **dass** das Verschlusselement (60) eine viren-, bakterien- und sporendichte Folie ist, die auf der Stirnfläche (21) und der Erhöhung (27) aufliegt und
- **dass** am Verschlusselement (60) ein Elastomerring (73) angeordnet ist, in den sich beim Aufsetzten der Kappe (81, 82) die Erhöhung (27) eindrückt.

11. Verschlusssystem für Behältnisse zur Aufbewahrung oder Verabreichung von flüssigen, pastösen oder pulverförmigen Stoffen, das ein folienartiges, viren-, bakterien- und sporendichtes Verschlusselement (60) aufweist, an dessen Rand ein Elastomerring (71, 74) festhaftend angeordnet ist,
- wobei das Verschlusselement (60) auf dem Behältnis (10, 50) im Bereich einer zu verschließenden Öffnung (12) auf einer die Behältnisöffnung (12) umgebende Stirnfläche (21) aufliegt und
- wobei der Elastomerring (74, 75) an einer radialen Außenkontur (32, 33) des Behältnisses (10, 50) federnd anliegt.

12. Verschlusssystem nach Anspruch 11,
**dadurch gekennzeichnet,**
**dass** der Durchmesser der radialen Außenkontur (32, 33) mit zunehmenden Abstand von der Stirnfläche (21) des Behältnisses (10, 50) - einen Hintergriff bildend - abnimmt.

13. Verschlusssystem nach Anspruch 11,
**dadurch gekennzeichnet,**
**dass** der Elastomerring (74, 75) durch einen ihn radial umfassenden Spannring (79) gegen die radiale Außenkontur (32, 33) gepresst wird.

## Claims

1. Closure system for containers used for storing or administering substances in the form of liquids, pastes or powders, which system is composed of a cap (81, 82), provided with a through-hole (85), and of a closure element (60), the cap (81, 82) holding the closure element (60) with a force fit and/or form fit on the container (10, 50), in the area of the opening (12) that is to be closed, by means of a catch element (37) present on the container (10, 50), **characterized in that**
- the end face (21) which surrounds the container opening (12), and on which the closure element (60) bears, comprises a depression (24, 25),
- the closure element (60) is a virus-proof, bacteria-proof and spore-proof film that lies on the end face (21) and, at least in some areas, over the depression (24, 25), and
- when the cap (81, 82) is fitted in place, an elastomer ring or adhesive ring (71, 72) is arranged between the closure element (60) and the depression (24, 25) and fills the depression (24, 25).

2. Closure system according to Claim 1,
**characterized in**
**that** the closure element (60) is a disc-shaped film.

3. Closure system according to Claim 2,
**characterized in**
**that** the film (60) has a material thickness of 50 to 300 micrometres.

4. Closure system according to Claim 2, **characterized in that** the film (60) is made from an aluminium alloy.

5. Closure system according to Claim 2,
**characterized in**
**that**, in the area of the opening (12) of the container (10, 50), the film (60) has, on its upper surface (61), a bead (62) that weakens the film (60).

6. Closure system according to Claim 1,
**characterized in**
**that** the depression (24) lies on the outer edge of the end face (21).

7. Closure system for containers used for storing or administering substances in the form of liquids, pastes or powders, which system is composed of a cap (83, 84), provided with a through-hole (85), and of a closure element (60), the cap (83, 84) holding the closure element (60) with a force fit and/or form fit on the container (10, 50), in the area of the opening (12) that is to be closed, by means of a catch element (37) present on the container (10, 50), **characterized in that**
- the closure element (60) is a virus-proof, bacteria-proof and spore-proof film, and
- at least one closed, elastic sealing lip (87, 88) is arranged bearing resiliently on the inner face (86) of the cap directed towards the closure element (60).

8. Closure system according to Claim 7,
**characterized in**
**that** an inner sealing lip (88) and an outer sealing lip (87) are arranged on the inner face (86) of the cap.

9. Closure system according to Claim 8,
**characterized in**
**that,** in at least one resilient sealing lip (88), the area in contact with the sealing film (60), and clamping the sealing film (60), is oriented towards the outside and its individual cross section encloses an angle of 30 to 60 angular degrees relative to the centre line of the container (10, 50).

10. Closure system for containers used for storing or administering substances in the form of liquids, pastes or powders, which system is composed of a cap (81, 82), provided with a through-hole (85), and of a closure element (60), the cap (81, 82) holding the closure element (60) with a force fit and/or form fit on the container (10, 50), in the area of the opening (12) that is to be closed, by means of a catch element (37) present on the container (10, 50), **characterized in that**
- the end face (21) which surrounds the container opening (12), and on which the closure element (60) bears, has an elevation (27) surrounding the container opening (12),
- the closure element (60) is a virus-proof, bacteria-proof and spore-proof film that lies on the end face (21) and on the elevation (27), and
- an elastomer ring (73) is arranged on the closure element (60), and the elevation (27) presses into said elastomer ring (73) when the cap (81, 82) is fitted in place.

11. Closure system for containers used for storing or administering substances in the form of liquids, pastes or powders, which system comprises a film-like, virus-proof, bacteria-proof and spore-proof closure element (60) on whose edge an elastomer ring (71, 74) is arranged in a fixed manner, and
- the closure element (60) bears on the container (10, 50), in the area of an opening (12) that is to be closed, namely on an end face (21) surrounding the container opening (12), and
- the elastomer ring (74, 75) bears resiliently on a radial outer contour (32, 33) of the container (10, 50).

12. Closure system according to Claim 11,
**characterized in**
**that** the diameter of the radial outer contour (32, 33) decreases as the distance from the end face (21) of the container (10, 50) increases, thereby forming an undercut.

13. Closure system according to Claim 11,
**characterized in**
**that** the elastomer ring (74, 75) is pressed against the radial outer contour (32, 33) by means of a clamping ring (79) that radially encloses said elastomer ring (74, 75).

## Revendications

1. Système de fermeture de récipients de conservation ou d'administration de substances liquides, pâteuses ou poudreuses, constitué d'un capuchon (81, 82) traversé par un trou (85) et d'un élément de fermeture (60), le capuchon (81, 82) maintenant mécaniquement et/ou géométriquement au moyen d'un élément d'encliquetage (37) prévu sur le récipient (10, 50) l'élément de fermeture (60) sur la partie du récipient (10, 50) occupée par l'ouverture (12) à fermer,
**caractérisé en ce que**
la surface frontale (21) qui entoure l'ouverture (12) du récipient et sur laquelle l'élément de fermeture (60) repose présente un creux (24, 25),
**en ce que** l'élément de fermeture (60) est une feuille étanche aux virus, aux bactéries et aux spores qui repose sur la surface frontale (21) et au moins en partie au-dessus du creux (24, 25) et
**en ce que** lorsque le capuchon (81, 82) est placé, une bague en élastomère ou en adhésif (71, 72) qui remplit le creux (24, 25) est placée entre l'élément de fermeture (60) et le creux (24, 25).

2. Système de fermeture selon la revendication 1, **caractérisé en ce que** l'élément de fermeture (60) est une feuille en forme de disque.

3. Système de fermeture selon la revendication 2,
**caractérisé en ce que** l'épaisseur de la feuille (60) est comprise entre 50 et 300 micromètres.

4. Système de fermeture selon la revendication 2, **caractérisé en ce que** la feuille (60) est constituée d'un alliage d'aluminium.

5. Système de fermeture selon la revendication 2, **caractérisé en ce que** dans la zone occupée par l'ouverture (12) du récipient (10, 50), la feuille (60) présente sur sa face supérieure (61) une moulure (62) qui affaiblit la feuille (60).

6. Système de fermeture selon la revendication 1, **caractérisé en ce que** le creux (24) repose sur le bord extérieur de la surface frontale (21).

7. Système de fermeture de récipients de conservation ou d'administration de substances liquides, pâteuses ou poudreuses, constitué d'un capuchon (83, 84) traversé par un trou (85) et d'un élément de fermeture (60), le capuchon (83, 84) maintenant mécaniquement et/ou géométriquement au moyen d'un élément d'encliquetage (37) prévu sur le récipient (10, 50) l'élément de fermeture (60) sur la partie du récipient (10, 50) occupée par l'ouverture (12) à fermer,
**caractérisé en ce que**
l'élément de fermeture (60) est une feuille étanche aux virus, aux bactéries et aux spores et
**en ce qu'**au moins une lèvre élastique fermée d'étanchéité (87, 88) et reposant élastiquement sur l'élément de fermeture (60) est disposée sur le côté intérieur (86) du capuchon, tourné vers l'élément de fermeture (60).

8. Système de fermeture selon la revendication 7, **caractérisé en ce qu'**une lèvre intérieure d'étanchéité (88) et une lèvre extérieure d'étanchéité (87) sont disposées sur le côté intérieur (86) du capuchon.

9. Système de fermeture selon la revendication 8, **caractérisé en ce que** sur au moins une lèvre élastique d'étanchéité (88), la partie en contact avec la feuille d'étanchéité (60) et tendant la feuille d'étanchéité (60) est orientée vers l'extérieur et forme ainsi en coupe transversale un angle de 30 à 60 degrés par rapport à l'axe central du récipient (10, 50).

10. Système de fermeture de récipients de conservation ou de distribution de substances liquides, pâteuses ou poudreuses, constitué d'un capuchon (81, 82) traversé par un trou (85) et d'un élément de fermeture (60), le capuchon (81, 82) maintenant mécaniquement et/ou géométriquement au moyen d'un élément d'encliquetage (37) prévu sur le récipient (10, 50) l'élément de fermeture (60) sur la partie du récipient (10, 50) occupée par l'ouverture (12) à fermer,
**caractérisé en ce que**
la surface frontale (21) qui entoure l'ouverture (12) du récipient et sur laquelle repose l'élément de fermeture (60) présente un rehaussement (27) qui entoure l'ouverture (12) du récipient,
**en ce que** l'élément de fermeture (60) est une feuille étanche aux virus, aux bactéries et aux spores qui repose sur la surface frontale (21) et sur le rehaussement (27) et
**en ce qu'**une bague en élastomère (73) dans laquelle s'enfonce le rehaussement (27) lorsque le capuchon (81, 82) est placé est disposée sur l'élément de fermeture (60).

11. Système de fermeture de récipients de conservation ou d'administration de substances liquides, pâteuses ou poudreuses, qui présente un élément de fermeture (60) en forme de feuille, étanche aux virus, aux bactéries et aux spores, et sur le bord duquel une bague en élastomère (71, 74) est disposée de manière adhérente, dans lequel l'élément de fermeture (60) repose sur le récipient (10, 50) dans la zone d'une ouverture (12) à fermer sur une surface frontale (21) qui entoure l'ouverture (12) du récipient et la bague en élastomère (74, 75) repose de manière élastique sur un contour extérieur radial (32, 33) du récipient (10, 50).

12. Système de fermeture selon la revendication 11, **caractérisé en ce que** le diamètre du contour extérieur radial (32, 33) diminue lorsque sa distance par rapport à la surface frontale (21) du récipient (10, 50) augmente, en formant une contre-dépouille.

13. Système de fermeture selon la revendication 11, **caractérisé en ce que** la bague en élastomère (74, 75) est comprimée contre le contour extérieur radial (32, 33) par une bague de serrage (79) qui l'entoure radialement.
